(19) Europäisches Patentamt
European Patent Office
Office européen des brevets

(11) EP 0 591 710 B1

(12) EUROPÄISCHE PATENTSCHRIFT

(45) Veröffentlichungstag und Bekanntmachung des Hinweises auf die Patenterteilung:
**26.06.1996 Patentblatt 1996/26**

(51) Int. Cl.[6]: **A61K 31/185**

(21) Anmeldenummer: **93114670.8**

(22) Anmeldetag: **13.09.1993**

(54) **Mesna-Injektionslösungen**

Injection solutions containing mesna

Solutions injectable contenant du mesna

(84) Benannte Vertragsstaaten:
**AT BE CH DE DK ES FR GB GR IE IT LI LU MC NL PT SE**

(30) Priorität: **08.10.1992 DE 4233842**

(43) Veröffentlichungstag der Anmeldung:
**13.04.1994 Patentblatt 1994/15**

(73) Patentinhaber: **ASTA Medica Aktiengesellschaft**
**D-01277 Dresden (DE)**

(72) Erfinder:
- **Engel, Jürgen, Prof. Dr.**
  **D-63755 Alzenau (DE)**
- **Wolf-Heuss, Elisabeth, Dr.**
  **D-74821 Mosbach (DE)**
- **Deger, Wolfgang, Dr.**
  **D-60389 Frankfurt (DE)**
- **Camuglia, Giancarlo**
  **D-60388 Frankfurt (DE)**
- **Sauerbier, Dieter**
  **D-33824 Werther (DE)**

(56) Entgegenhaltungen:
**EP-A- 0 198 542** **EP-A- 0 334 083**

- **ANNALS OF ONCOLOGY Bd. 1, Nr. 5, 1990 Seiten 365 - 368 T.CERNY ET AL. 'Bioavailability of subcutaneous ifosfamide and feasibility of continuous outpatient application in cancer patients'**
- **EUR. J. CANCER Bd. 27, Nr. 10, 1991 Seiten 1243 - 1247 O.R. LEEUWENKAMP ET AL. 'Reaction kinetics of Cisplatin and its monoaquated species with the modulating agents (di)mesna and thiosulphate'**
- **J. MED. CHEMISTRY Bd. 30, Nr. 2, 1987 Seiten 395 - 399 C-H. KWON ET AL. 'Activation mechanisms of mafosfamide and the role of thiols in cyclophosphamide metabolism'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 591 710 B1

Printed by Rank Xerox (UK) Business Services
2.12.4/3.4

## Beschreibung

Der chemische Name für den Wirkstoff Mesna ist Natrium-2-mercaptoethansulfonat. (Formel I)

$$HS\text{-}CH_2\text{-}CH_2\text{-}SO_3Na \qquad \text{Formel I}$$

Mesna schützt beispielsweise die harnableitenden Organe bei der Therapie von Tumorerkrankungen mit Ifosfamid. Außerdem wird Mesna seit langer Zeit bereits als Mucolytikum eingesetzt.

Mesna ist ein weißes, hygroskopisches Pulver von charakteristischem Geruch. Die Substanz ist stark oxidationsempfindlich und setzt sich bei Kontakt mit Sauerstoff, insbesondere in feuchter Atmosphäre schnell zu Dimesna um. (Formel II)

$$NaSO_3\text{-}CH_2\text{-}CH_2\text{-}S\text{-}S\text{-}CH_2\text{-}CH_2\text{-}SO_3Na \qquad \text{Formel II}$$

Mesna wird bisher oral, parenteral und inhalativ appliziert. Bei allen verwendeten Darreichungsformen handelt es sich um flüssige Zubereitungen, die in Ampullen- oder Trinkampullenform angeboten werden. Da Mesna sehr oxidationsempfindlich ist und in Gegenwart von Sauerstoff zu dem schwer resorbierbaren Dimensa reagiert, muß die wässrige Lösung vor Sauerstoff geschützt sein. Zu diesem Zweck wird die Lösung in Glasampullen eingeschweißt.
Die Lösung für parenterale Application wird in Ampullen zu 2 ml, 4 ml und 10 ml angeboten, die 10 % Mesna enthalten. Mesna wird zur Vermeidung urotoxischer Erscheinungen bei der Therapie mit Ifosfamid eingesetzt.

Mesna wird im allgemeinen gleichzeitigg mit Ifosfamid sowie nach 4 und nach 8 Stunden jeweils in einer Dosis, die 20 % der Ifosfamid-Dosis entspricht, intravenös appliziert. Die Ifosfamid-Dosis kann zwischen 1g/m$^2$ bis 8 g/m$^2$ Körperoberfläche liegen. Die entsprechenden Mesna-Dosen liegen daher in einem sehr bereiten Spektrum. Es wurde daher der Wunsch nach Mehrdosenbehältnissen vorgebracht, aus denen jede beliebige Menge Injektionslösung entnommen werden kann und keine Verluste, die bei angebrochenen Ampullen entstehen, zu verzeichnen sind. Außerdem sollten diese Injektionsflaschen ein größeres Volumen an Lösung enthalten.

Zum Schutz vor mikrobieller Kontamination müssen laut DAB 9 Mehrdosenbehälter konserviert werden. Die in Ampullen abgefüllte Injektionslösung ist sehr stabil. Diese Rezeptur wurde daher übernommen und lediglich ein Konservierungsmittel, nämlich Benzylalkohol, der für wässrige Systeme, die parenteral appliziert werden, weltweit eingesetzt wird, zugesetzt.
Der pH-Wert dieser Lösung wird mit Natronlauge auf 6,5 - 8,5 eingestellt. Außerdem enthält die Lösung ein Komplexierungsmittel, da Mesna mit Schwermetallionen gefärbte Komplexe bildet.

Die Stabilitätslagerung zeigte jedoch, daß diese Lösungen teilweise instabil waren.
Das Zersetzungsprodukt wurde als Acetal von Mesna mit Benzaldehyd (Formel III)

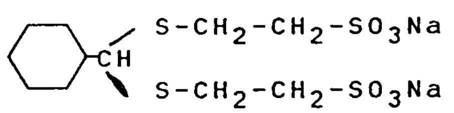

Formel III

identifiziert. Benzaldehyd entsteht in der Formulierung durch Oxidation des verwendeten Konservierungsmittels Benzylalkohol.

Überraschenderweise wurde nun gefunden, daß dieses Zersetzungsprodukt nur bei pH-Werten < 7,5 entsteht und bei einer pH-Einstellung 7,5 - 8,5 nicht gebildet wird.

Die Erfindung betrifft somit eine Injektionslösung, die, bezogen auf 1 Teil Mesna
beispielsweise 0,00002 - 2,0 Gewichtsteile
vorzugsweise 0,0001 - 1,0 Gewichtsteile
insbesondere 0,0002 - 0,7 Gewichtsanteile NaEDTA
als Komplexierungsmittel und
beispielsweise 0,0002 - 2,0 Gewichtsanteile
vorzugsweise 0,0003 - 1,7 Gewichtsanteile
insbesondere 0,0004 - 1,4 Gewichtsanteile
Natriumhydroxid zur pH-Einstellung und
beispielsweie 0,001 - 30,0 Gewichtsanteile
vorzugsweise 0,01 - 20,0 Gewichtsanteile
insbesondere 0,018 - 12,0 Gewichtsanteile
Benzylalkohol als Konservierungsmittel enthält.

Die Gewichtsmenge an Mesna in einer solchen Injektionslösung beträgt im allgemeinen 1 mg pro ml bis 540 mg/ml. Die Lösungen können direkt i.v. appliziert werden oder als Infusionslösungen oder Konzentrate als Zusatz zu Infusionen konzipiert werden.
Die entsprechenden Volumina liegen zwischen 0,5 ml und 100 ml. Als Lösungsmittel wird Wasser für Injektionszwecke eingesetzt.

Neben den oben aufgeführten Hilfsstoffen können auch eingesetzt werden:

Komplexierungsmittel:
Zum Beispiel das Calcium-Dinatriumsalz der Edetinsäure, Citronensäure, Weinsäure oder Salze der Orthophosphorsäure.
Zur pH-Einstellung eignen sich zum Beispiel
Natriumhydroxid, Kaliumhydroxid, Trometamol, Natriumacetat, Trinatriumcitrat, Natriummonohydrogenphosphat, Kaliummonohydrogenphosphat.
Konservierungsmittel:
Zum Beispiel p-Hydroxybenzoesäureester, m-Kresol, Organomercuriverbindungen, Chlorbutanol, quartäre Ammoniumverbindungen.

Die Konservierungsmittel können auch in Kombination mit Benzylalkohol eingesetzt werden.

Zur Herstellung der Lösung werden 80-90 %, vorzugsweise 85 % der erforderlichen Wassermenge (sauerstoffarm) vorgelegt und unter Rühren und ständiger Stickstoffbegasung Natriumedetat, Benzylalkohol und Mesna gelöst. Nach vollständiger Auflösung wird durch Zugabe von 10N Natronlauge ein pH-Wert von 8,0 eingestellt. Diese Lösung wird mit Wasser (sauerstoffarm) auf das Endvolumen aufgefüllt.

Die so erhaltene Lösung wird durch Filtration über hierfür übliche, keimdichte Filter sterilisiert und dann in entsprechende Behälter für Injektionspräparate abgefüllt. Die Lösung wird während und nach der Filtration mit Stickstoff überschichtet. Die Aufbewahrungszeit bis zur Abfüllung in die Injektionsbehälter soll einschließlich der Zeit der Lösungsherstellung eine Zeit von 5 Stunden nicht überschreiten.

Zur Sterilisation werden übliche keimdichte Filter, beispielsweise übliche Bakerienfilter mit einer Porengröße von 0,2 µm verwendet. Die verwendeten Glasgefäße werden vorher in üblicher Weise sterilisiert. Das verwendete Injektionswasser muß steril und pyrogenfrei sein und den Anforderungen des Deutschen Arzneibuches 10. Ausgabe, 1991 entsprechen. Als Injektionsgefäß werden zweckmäßig solche aus Röhrenglas beziehungsweise Hüttenglas der II. hydrolytischen Klasse (beispielsweise 10R, 30R, 50H) siehe hierzu Deutsches Arzneibuch 10. Ausgabe 1991, VI.2.1. und DIN-Normen 58366 Teil 1 und Teil 5) eingesetzt.
Weiterhin sollen die Injektionsgefäße sowie die weiteren Hilfsstoffe, wie Gummistopfen und Bördelkappen, den Anforderungen der DIN-Normen 58366, Teil 2 und Teil 3 sowie 58367, Teil 1 entsprechen.

Die Lösungsmengen in den jeweiligen Behältern liegen pro Behälter zwischen 4 ml und 100 ml, vorzugsweise zwischen 10 ml und 75 ml, insbesondere zwischen 40 ml und 60 ml. Die Mesna-Menge pro Glasgefäß beträt beispielsweise 4 mg bis 50 g, vorzugsweise 1 g bis 7,5 g, insbesondere 4 g - 6 g.
Nach Abfüllung der Lösung unter aseptischen Bedingungen werden die Flaschen mit Stickstoff zur Verdrängung des Sauerstoffs begast und mit Injektionsstopfen verschlossen. Die gefüllten Injektionsflaschen werden im Dampfsterilisator sterilisiert.

Folgende Beispiele veranschaulichen die Erfindung:

Beispiel 1

Injektionsflasche mit 50 ml einer 10 %igen Mesna Injektionslösung

Zusammensetzung

| | |
|---|---|
| Mesna (INN) | 5000,0 mg |
| Benzylalkohol | 520,0 mg |
| Natriumedetat | 12,5 mg |
| Natriumhydroxid | 10,0 - 70,0 mg 1) 2) |
| Wasser für Injektionszwecke 50,0 ml ≙ 52,5 g | |

1) Dient der pH-Wert-Einstellung und ist daher variabel.
2) Eingesetzt als 10n Natronlauge 25-175 x $10^{-3}$ ml 3)
3) Mehr- oder Mindereinsatz wird durch Wasser für Injektionszwecke ausgeglichen.

Die Dichte der Lösung beträgt 1,05 g/ml.

Herstellung der Lösung

In einem geeigneten Gefäß werden 85 % Injektionswassermenge (sauerstoffarm) vorgelegt und unter Rühren und ständiger Stickstoffbegasung die entsprechenden Mengen Natriumedetat, Benzylalkohol und Mesna aufgelöst. Durch Zugabe von 10N Natronlauge wird ein pH-Wert von 8,0 eingestellt und mit Waser für Injektionszwecke (sauerstoffarm) auf das Endvolumen aufgefüllt.
Das Wasser für Injektionszwecke darf max. 1,5 ppm Sauerstoff bei 20°C enthalten. Als Inprozeßkontrollen werden pH-Wert, Dichte und Berechnungsindex($n_D^{20}$ 1,34-1,36) bestimmt.
Die Sterilisation der Lösung erfolgt durch Filtration über Membranfilter 0,2 µm. Die Lösung wird während und nach der Filtration mit Stickstoff überschichtet. Als übliche keimdichte Filter werden z.B. Sartorius SM 11107 oder SM 11307 oder Pall Filter NRP verwendet. Die Lösung wird unter Vermeidung partikulärer und bakterieller Kontamination bis zu Abfüllung aufbewahrt. Eine Lagerung bei Raumtemperatur (20° -22°C) soll 4 Stunden nicht überschreiten. Zur Sterilfiltration können zusätzlich übliche Vorfilter (zum Beispiel Sartorius SM 13400 oder Pall LPA) zum Schutz der Sterilfilter eingesetzt werden.

Reinigung der Injektionsflaschen

Als Behältnisse werden Injektionsflaschen DIN 50H/II, farblos, 50 ml eingesetzt. Sie werden mit demineralisiertem Waser warm und kalt und Luft gespült. Sämtliche Reinigungsmedien werden durch Filtration von Schwebstoffen befreit. Unter Vermeidung von Rekontamination durch Partikel aus der Luft werden die Flaschen mittels Heißluft getrocknet und sterilisiert (diskontiunuierlich bei 180°C, 2 Stunden, kontinuierlich bei 350°C 10 Minuten).

Die Reinigung der Gummistopfen (zum Beispiel Gummistopfen Helvoet FM 157/1 grau), mit denen die Injektionsflaschen verschlossen werden, erfolgt unter Verwendung von demineralisiertem Wasser und beispielsweise einem Reinigungsmittel, bestehend aus nichtionogenen Tensiden und Phosphorsäureestern in wäßriger Lösung (zum Beispiel MB 70, Fa. Huber, Freiburg).
Die gereinigten Stopfen werden unter Verwendung von Wasser für Injektionzwecke oder filtriertem demineralisiertem Wasser faser- und flusenfrei gespült. Die so gereinigten Stopfen werden dann mittels Dampf sterilisiert.
Die so gereinigten und sterilisierten Injektionsflaschen werden nun aseptisch mit Stickstoff zur Verdrängung des Luftsauerstoffs begast, mit 52 ml Mesnalösung gefüllt und nachbegast. Schließlich werden die Stopfen aufgesetzt und mit Bördelkappen gesichert. Durch Nachwiegen des Inhalts wird das Füllvolumen statistisch überwacht. Sollfüllmenge 52 ml = 54,6 g. Außerdem wird der Restsauerstoffgehalt im Kopfraum der Injektionsflasche überwacht.
Die gefüllten Injektionsflaschen werden im Dampfsterilisator sterilisiert (mindestens 120° / 20 Minuten).
Die Injektionsflaschen werden auf Verschlußfehler, äußere Fehler, Klarheit und partikulare Verunreinigungen kontrolliert.

Beispiel 2

Injektionsflasche mit 50 ml eines 50 %igen Konzentrates (G/V) als Zusatz zu Infusionslösungen.

Zusammensetzung:

| | |
|---|---|
| Mesna | 25,000 g |
| Benzylalkohol | 0,520 g |
| Na EDTA | 0,0125 g |
| Natriumhydroxid | 0,05 - 0,35 g |
| Wasser f. Injektionszwecke ad 50,000 ml = 61,0 g | |

Die Dichte des Konzentrates beträgt 1,22 g/ml 20°C. Die Herstellung und Sterilfiltration der Lösung, Reinigung und Sterilisation der Flaschen und Gummistopfen, Abfüllung, Verschließen der Flaschen und Endsterilisation erfolgen entsprechend Beispiel 1.

## Patentansprüche

1. Mesna-Injektionslösung,
dadurch gekennzeichnet, daß
der pH-Wert auf einen Wert über 7,5 eingestellt wird.

2. Verfahren zur Herstellung einer Mesna-Injektionslösung,
dadurch gekennzeichnet,
daß man eine Mesna-Lösung durch Zugabe von alkalischen Substanzen auf einen pH-Wert von über 7,5 einstellt.

3. Verwendung der Mesna-Injektionslösung gemäß Anspruch 1 zur Herstellung eines Arzneimittels zur Vermeidung der urotoxischen Nebenwirkungen der Therapie durch Oxazaphosphorine.

4. Arzneimittel zur Vermeidung der Oxazaphosphorin-Nebenwirkung,
dadurch gekennzeichnet,
daß aus einer Mesna-Injektionslösung mit einem pH-Wert, der größer als 7,5 ist, besteht.

## Claims

1. Mesna injection solution,
characterised in that
the pH value is adjusted to a value of above 7.5.

2. Process for the production of a mesna injection solution,
characterised in that
a mesna solution is adjusted to a pH value of above 7.5 by the addition of alkaline substances.

3. Use of the mesna injection solution according to claim 1 for the production of a pharmaceutical preparation to prevent the urotoxic side effects of treatment with oxazaphosphorines.

4. Pharmaceutical preparation to prevent the side effect of oxazaphosphorine,
characterised in that
it consists of a mesna injection solution with a pH value which is greater than 7.5.

## Revendications

1. Solution injectable de mesna, caractérisée en ce que la valeur de pH est ajustée à une valeur supérieure à 7,5.

2. Procédé de préparation d'une solution injectable de mesna, caractérisé en ce qu'on ajuste une solution de mesna par addition de substances alcalines à une valeur de pH de plus de 7,5.

3. Utilisation de la solution injectable de mesna selon la revendication 1 pour la préparation d'un médicament destiné à éviter les effets secondaires urotoxiques de la thérapie par oxazaphosphorine.

**4.** Médicament pour éviter l'effet secondaire de la oxazaphosphorine, caractérisé en ce qu'il se compose d'une solution injectable de mesina ayant une valeur de pH, qui est supérieure à 7,5.